(19) Europäisches Patentamt
European Patent Office
Office européen des brevets

(11) EP 4 732 849 A1

(12) **EUROPEAN PATENT APPLICATION**
published in accordance with Art. 153(4) EPC

(43) Date of publication:
**29.04.2026 Bulletin 2026/18**

(21) Application number: **24825994.7**

(22) Date of filing: **21.06.2024**

(51) International Patent Classification (IPC):
***A61K 47/64*** *(2017.01)* ***A61K 31/7088*** *(2006.01)*
***A61K 47/26*** *(2006.01)* ***A61K 47/34*** *(2017.01)*
***A61K 47/36*** *(2006.01)* ***A61K 47/42*** *(2017.01)*
***A61K 47/61*** *(2017.01)* ***A61P 43/00*** *(2006.01)*

(52) Cooperative Patent Classification (CPC):
**A61K 31/7088; A61K 47/26; A61K 47/34; A61K 47/36; A61K 47/42; A61K 47/61; A61K 47/64; A61P 43/00**

(86) International application number:
**PCT/JP2024/022479**

(87) International publication number:
**WO 2024/262593 (26.12.2024 Gazette 2024/52)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **22.06.2023 JP 2023102506**

(71) Applicant: **Kawasaki Institute of Industrial Promotion**
**Kawasaki-shi, Kanagawa 212-0013 (JP)**

(72) Inventors:
- **KATAOKA Kazunori**
  **Kawasaki-shi Kanagawa 212-0013 (JP)**
- **TOH Kazuko**
  **Kawasaki-shi Kanagawa 212-0013 (JP)**
- **KINOH Hiroaki**
  **Kawasaki-shi Kanagawa 212-0013 (JP)**

(74) Representative: **Klöckner, Christoph**
**df-mp Patentanwälte Rechtsanwälte PartG mbB**
**Theatinerstraße 16**
**80333 München (DE)**

Remarks:
The complete document including Reference Table(s) and the Sequence Listing(s) can be downloaded from the EPO website

(54) **METHOD FOR IMPROVING STABILITY OF NUCLEIC ACID IN BLOOD AND IMPROVING TARGETING EFFICIENCY OF NUCLEIC ACID**

(57) The present disclosure provides a technology concerning stabilization and targeting of nucleic acids. According to the present disclosure, there is provided a composition comprising a complex of a nucleic acid (ligand-linked nucleic acid) linked to a ligand for a target and a block copolymer having a bulky non-ionic hydrophilic polymer block and a polycation block.

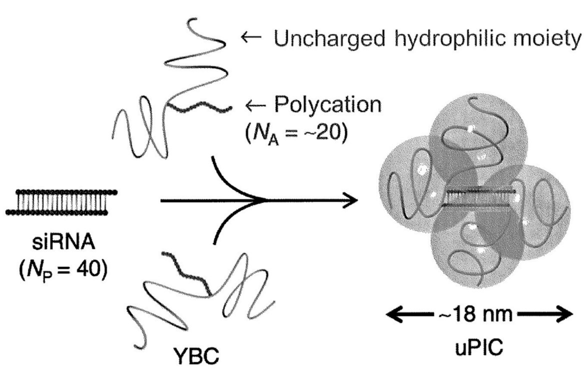


FIG. 1

EP 4 732 849 A1

## Description

TECHNICAL FIELD

The present disclosure relates to a technology concerning stabilization and targeting of nucleic acids.

BACKGROUND ART

Nucleic acids can be advantageously used for expression or suppression of various physiological functions in cells. However, there is a need for a technology to stably deliver nucleic acids to target cells *in vivo.* A technology for encapsulating nucleic acids in lipid nanoparticles and delivering them into cells has been established (Patent Document 1). Viruses such as adeno-associated viruses are used to store nucleic acids in viral particles and deliver them into cells (Patent Document 2). Technologies for delivering nucleic acids to cells by a polyion complex of a nucleic acid and a polycation have been developed (Patent Documents 3 and 4). Patent Document 4 discloses a composition containing a complex (unit structure) of a nucleic acid and a polycation, which is electrically neutralized. Patent Document 5 discloses reducing the clearance of a drug used in combination with an excess amount of a polyvalent cation or prior administration of a polyvalent cation. Patent Document 6 discloses that an excess amount of a cationic polymer or prior administration of a polyvalent cation contributes to the improvement of blood stability of mRNA used in combination.

PRIOR ART DOCUMENTS

PATENT DOCUMENTS

Patent Document 1: US8058069B
Patent Document 2: WO2017/212019
Patent Document 3: WO2019/044937
Patent Document 4: WO2013/162041
Patent Document 5: WO2019/176916
Patent Document 6: WO2018/216792

SUMMARY OF THE INVENTION

The present disclosure provides a technology concerning stabilization and targeting of nucleic acids. According to the present disclosure, there is provided a composition comprising a complex of a nucleic acid linked to a ligand for a target (ligand-linked nucleic acid) and a block copolymer having a bulky non-ionic hydrophilic polymer block and a polycation block. In conventional methods, a ligand that binds to a target is linked to the surface of a particle, and the nucleic acid is delivered to a cell expressing the target by utilizing the action of the ligand. On the other hand, in an assembly (complex; also referred to as uPIC) of one molecule of nucleic acid and two molecules of polycation, the surface of the particle is covered with the non-ionic hydrophilic polymer block and the ligand is not exposed, so it was considered that targeting to a specific cell by the ligand is not possible. However, in the uPIC of the present disclosure, surprisingly, it could be effectively used for targeting to a specific cell.

According to the present disclosure, for example, the nucleic acid and the block copolymer form a complex, and can improve the blood retention of the nucleic acid (for example, see FIG. 2). According to the present disclosure, also, the complex of the ligand-linked nucleic acid and the block copolymer can deliver the nucleic acid into a target cell (for example, see FIG. 3B). According to the present disclosure, further, by using siRNA as the nucleic acid, the nucleic acid is delivered into the target cell and can effectively silence the target nucleic acid (see FIG. 4). According to the present disclosure, furthermore, the cells to be delivered can be changed according to the type of the ligand (see FIGS. 6 and 7). Thus, according to the present disclosure, a technology for stabilizing and targeting a nucleic acid can be provided.

According to the present disclosure, the following inventions are provided as an example.

(1) A composition comprising a complex of a polycation and a nucleic acid,

wherein the nucleic acid is covalently linked to a ligand for a target,
the polycation is in the form of a block copolymer of a branched non-ionic hydrophilic polymer block and a polycation block,
the branched non-ionic hydrophilic polymer comprises at least one branch and at least two non-ionic hydrophilic

polymers extending from the said branch, and
relative to the absence of the polycation, the composition improves blood retention of the nucleic acid, and, relative to a complex comprising a nucleic acid not linked to the ligand, improves migration ability toward the target.

(2) The composition according to (1) above, wherein a charge ratio (charge of polycation / charge of nucleic acid) of the polycation to the nucleic acid contained in the composition is 0.1 to 5 (for example, 0.1 or more and less than 1.0, for example, 0.3 to 0.7).
(3) The composition according to (1) or (2) above, wherein each non-ionic hydrophilic polymer has a number-average molecular weight of 10 kDa or more.
(4) The composition according to any one of (1) to (3) above, wherein each non-ionic hydrophilic polymer is a polyalkylene glycol.
(5) The composition according to any one of (1) to (4) above, wherein the polycation block has a number-average degree of polymerization of 15 to 30.
(6) The composition according to any one of (1) to (5) above, wherein the polycation block is poly-L-lysine or poly-L-ornithine.
(7) The composition according to any one of (1) to (6) above, wherein the ligand is a sugar or a polysaccharide.
(8) The composition according to any one of (1) to (7) above, wherein the polycation is a block copolymer comprising a branched non-ionic hydrophilic polymer that includes polyethylene glycol (PEG) each having a number-average molecular weight of 10 kDa or more, and a polycation block comprising poly-L-lysine or poly-L-ornithine, and wherein the polycation block has a number-average degree of polymerization of 15 to 30.
(9) The composition according to any one of (1) to (8) above, wherein the ligand is an RGD peptide or a cRGD peptide.
(21) The composition according to any one of the above, wherein the nucleic acid is a double-stranded nucleic acid.
(22) The composition according to (21) above, wherein the nucleic acid is siRNA or shRNA.
(23) The composition according to (21) or (22) above, wherein the nucleic acid has a length of (n) mer, and the number-average degree of polymerization of the polycation block is (n) $\times$ 0.8 to 1.25 {wherein (n) represents a natural number}.
(41) The composition according to any one of the above, wherein the nucleic acid is a single-stranded nucleic acid.
(42) The composition according to (21) or (22) above, wherein the nucleic acid has a length of (n) mer, and the number-average degree of polymerization of the polycation block is (n) $\times$ 0.4 to 0.6 {wherein (n) represents a natural number}.
(61) The composition according to any one of the above, wherein, in the complex consisting of the polycation and the nucleic acid in the composition, the proportion of a complex consisting of one molecule of polycation and one molecule of nucleic acid is 1% or more.
(62) The composition according to any one of the above, wherein, in the complex consisting of the polycation and the nucleic acid in the composition, the proportion of a complex consisting of two molecules of polycation and one molecule of nucleic acid is 90% or less.
(63) The composition according to (61) above, wherein, in the complex consisting of the polycation and the nucleic acid in the composition, the proportion of a complex consisting of two molecules of polycation and one molecule of nucleic acid is 90% or less.
(81) The composition according to any one of the above, for use in delivering a nucleic acid to a target tissue or cell.
(82) The composition according to any one of the above, wherein the target tissue is liver, and the ligand is capable of binding to a target in the liver.
(83) The composition according to any one of the above, wherein the target cell is a hepatic stellate cell, and the ligand is capable of binding to a target in the hepatic stellate cell.
(101) A method of administering a nucleic acid to a subject, comprising administering any one of the above compositions to the subject.
(102) The method according to (101) above, wherein the administration is intravenous administration.
(201) The composition described above, for use in the method described above.
(202) The complex defined in any one of the above, for use in the method described above.
(301) Use of the nucleic acid, ligand, or polycation, or complex defined in any one of the above in the manufacture of a composition for use in the method described above.

BRIEF DESCRIPTION OF THE DRAWINGS

[FIG. 1] FIG. 1 shows that by mixing one molecule of nucleic acid (siRNA having a length of about 20 mer as an example) and two molecules of polycation (polycation having two non-ionic hydrophilic polymer blocks and having a degree of polymerization of 20 as an example), an assembly (complex; also referred to as uPIC) of one molecule of

nucleic acid and two molecules of polycation is obtained. The diameter of the obtained uPIC is estimated to be about 18 nm. Since the non-ionic hydrophilic polymer block is bulky and forms steric hindrance, it is not easy for three or more molecules of polycation to simultaneously bind to one molecule of nucleic acid.

[FIG. 2] FIG. 2 shows the evaluation results of blood retention of the ligand-linked unit polyion complex (uPIC) of the present disclosure. GalNAc was used as the ligand, and the case with linkage to the ligand (right panel) and the case without linkage to the ligand (left panel) are compared. NP indicates the number of side chain amino groups in ornithine (N) / the number of phosphates in nucleic acid (P) (N/P ratio) (the same applies hereinafter). Naked indicates the evaluation result of blood retention of the ligand-linked nucleic acid in the absence of polycation (the same applies hereinafter).

[FIG. 3A] FIG. 3A shows the distribution of uPIC in the liver of a mouse after administration of the ligand-linked uPIC of the present disclosure. GalNAc-linked uPIC accumulates in the liver and migrates to the liver parenchyma at 1 hour, 3 hours, and 9 hours after administration.

[FIG. 3B] FIG. 3B is a graph showing the accumulation amount of the ligand-linked uPIC of the present disclosure in liver parenchymal cells.

[FIG. 4] FIG. 4 shows the knockdown effect of a target nucleic acid by the ligand-linked uPIC of the present disclosure. GalNAc was used as the ligand, and the case with linkage to the ligand (right panel) and the case without linkage to the ligand (left panel) are compared.

[FIG. 5] FIG. 5 shows the evaluation results of blood retention of the ligand-linked unit polyion complex (uPIC) of the present disclosure. cRGD peptide was used as the ligand, and the case with linkage to the ligand (right panel) and the case without linkage to the ligand (left panel) are compared.

[FIG. 6] FIG. 6 shows the distribution of uPIC in the liver of a mouse after administration of the ligand-linked uPIC of the present disclosure. GalNAc-linked uPIC accumulates in the liver and migrates to the liver parenchyma at 1 hour, 3 hours, and 9 hours after administration. cRGD-linked uPIC accumulates in the liver at 1 hour, 3 hours, and 9 hours after administration, but shows a distribution different from that of GalNAc-linked uPIC.

[FIG. 7] FIG. 7 shows the distribution of uPIC in the liver of a mouse after administration of the ligand-linked uPIC of the present disclosure. Co-localization of the stained image of α-smooth muscle actin (αSMA) and cRGD-linked uPIC is shown.

## DESCRIPTION OF EMBODIMENTS

<Definition of Terms>

In the present specification, defined terms have the meanings as defined. Undefined terms have meanings generally used in this technical field. The singular form does not exclude the plural. "Comprising" means that elements other than those specified may be included, and "consisting of' means that elements other than those specified are not substantially included (for example, inclusion of the element unavoidable in manufacture is merely included, or included only in an amount that does not substantially affect the function, or the element is included only below the detection limit) or are not included.

In the present specification, a "subject" may be an animal. Examples of the animal include vertebrates, for example, mammals and birds. Examples of mammals include primates such as humans, rodents such as mice and rats, and domestic mammals such as cows, horses, sheep, donkeys, sheep, goats, llamas, and camels. Examples of birds include chickens.

In the present specification, a "target tissue" is an organ or tissue in the body of a subject. The target can be a vascular endothelial cell of a tissue blood vessel of the target tissue or a molecule expressed on the cell surface. The target may be the liver.

In the present specification, "nucleic acid" includes deoxyribonucleic acid (DNA), ribonucleic acid (RNA), and modified nucleic acids thereof. Examples of the nucleic acid include, but are not particularly limited to, nucleic acids containing only DNA, nucleic acids containing only RNA, nucleic acids containing only DNA and RNA, nucleic acids containing only modified nucleic acids, nucleic acids containing DNA and modified nucleic acids, nucleic acids containing RNA and modified nucleic acids, nucleic acids containing DNA, RNA and modified nucleic acids, and the like. Examples of the nucleic acid also include mRNA, antisense nucleic acids targeting RNA (e.g., antisense oligo), siRNA, shRNA, microRNA, decoy nucleic acids that bind to transcription factors to suppress transcription steps, aptamers that bind to proteins to inhibit functions, and CpG oligodeoxynucleotides that act on Toll-like receptor 9 to activate the immune system. The base length of the nucleic acid is not particularly limited, but is, for example, about 10 to about 80 bases, about 15 to about 50 bases, or about 20 to about 30 bases. The length of siRNA may be, for example, about 20 to about 30 bases, about 20 to about 24 bases, or about 21 to about 23 bases. The nucleic acid may contain a modified nucleic acid in its base sequence. That is, the nucleic acid may be a nucleic acid such as DNA, RNA, a hybrid of DNA and RNA, and a nucleic acid containing a modified nucleic acid (gapmer and mixmer). A gapmer is a single-stranded nucleic acid having a DNA moiety

and an RNA or modified nucleic acid moiety, wherein the RNA or modified nucleic acid moiety includes single-stranded nucleic acids located at both ends of the DNA moiety. The gapmer may be, for example, a double-stranded nucleic acid. A mixmer is a nucleic acid containing antisense containing different types of nucleic acids.

In the present specification, "modified nucleic acid" is a derivative of DNA or RNA, and may be DNA or RNA modified for the purpose of enhancement of hybridization ability, stability against degradation, thermal stability, and the like. Examples of the modified nucleic acid include, but are not particularly limited to, fluorescence dye-modified nucleic acids, biotinylated nucleic acids, and nucleic acids into which a cholesteryl group is introduced. RNA may be subjected to 2'-O-methyl modification, 2'-fluoro modification, or 2'-methoxyethyl (MOE) modification on the base to enhance stability, and the phosphodiester bond of the nucleic acid backbone may be replaced with a phosphorothioate bond. Examples of the modified nucleic acid include cross-linked nucleic acids. Examples of such artificial nucleic acids include locked nucleic acid (LNA), which is a cross-linked DNA in which an oxygen atom at the 2'-position and a carbon atom at the 4'-position are cross-linked via methylene, ENA, in which an oxygen atom at the 2'-position and a carbon atom at the 4'-position are cross-linked via ethylene, cross-linked nucleic acids (BNA) such as BNACOC, in which an oxygen atom at the 2'-position and a carbon atom at the 4'-position are cross-linked via $-CH_2OCH_2-$, and BNANC, in which an oxygen atom at the 2'-position and a carbon atom at the 4'-position are cross-linked via $-NR-CH_2-$ {wherein R is a methyl or a hydrogen atom}, cMOE, in which an oxygen atom at the 2'-position and a carbon atom at the 4'-position are cross-linked via $-CH_2(OCH_3)-$, cEt, in which an oxygen atom at the 2'-position and a carbon atom at the 4'-position are cross-linked via $-CH_2(CH_3)-$, AmNA, in which carbon atoms at the 2'-position and the 4'-position are cross-linked via an amide, scpBNA, in which an oxygen atom at the 2'-position and a carbon atom at the 4'-position are cross-linked via methylene and a cyclopropane is formed at the 6'-position, peptide nucleic acids (PNA), whose main chain is a polymer in which N-(2-aminoethyl)glycine is amide-bonded instead of deoxyribose or ribose, and morpholino oligos, in which bases are linked by a morpholine ring (for example, US 9,469,664B, which is incorporated herein by reference in its entirety). The modified nucleic acid may have acidic properties, but need not be acidic. mRNA includes mRNA containing a modified nucleoside. Examples of the mRNA containing a modified nucleoside include modified nucleosides described in US 8,278,036B, which is incorporated herein by reference in its entirety. Examples of the modified nucleoside include pseudouridine, which is known as a modified nucleoside for *in vivo* expression of mRNA. A modified nucleoside can replace an unmodified nucleoside. Pseudouridines include, for example, 1-methyl-3-(amino-5-carboxypropyl)pseudouridine ($m^1acp^3\Psi$), 1-methylpseudouridine (m1Ψ), 2'-O-methylpseudouridine (Ψm), 5-methyldihydrouridine (m5D), 3-methylpseudouridine (m3Ψ), and the like, which can replace uridine. The modified mRNA preferably contains pseudouridine (more preferably m1Ψ), and may preferably further contain 5-methylcytidine.

In the present specification, "siRNA" is a small interfering RNA and is a type of nucleic acid. siRNA is used to control the expression of a target gene by utilizing the RNA interference phenomenon. The length of siRNA may be, for example, about 20 to about 30 bases, about 20 to about 24 bases, or about 21 to about 23 bases. siRNA forms a double-stranded RNA. It is known that short hairpin RNA (shRNA) in which double strands of siRNA are linked by a hairpin controls the expression of a target gene similarly to siRNA. siRNA and shRNA are molecules consisting of RNA, but the nucleic acid may be a nucleic acid such as a hybrid of DNA and RNA having a sequence corresponding to the siRNA or shRNA, and a nucleic acid containing a modified nucleic acid (gapmer and mixmer).

In the present specification, antisense oligo (ASO) is a fragment of DNA or RNA capable of controlling gene expression. Antisense oligo has a sequence complementary to messenger RNA (mRNA), binds to mRNA, and inhibits translation of protein from mRNA. Antisense oligo has a length of about 12 to 40 mer, and may be usually about 12 to 28 mer. By making it a moderate length, excessive non-specific hybridization can be prevented, and the possibility of reduced uptake into cells can be reduced.

<Composition of the Present Disclosure>

According to the present disclosure, a complex of a polycation and a nucleic acid, and a composition containing the complex are provided.

The polycation may be a block copolymer containing a non-ionic hydrophilic polymer block and a polycation block. The non-ionic hydrophilic polymer block is bulky and can protect the nucleic acid from degradation or the like as a protective barrier for the bound nucleic acid. In addition, the non-ionic hydrophilic polymer block has biocompatibility and can improve the blood retention of the nucleic acid.

The non-ionic hydrophilic polymer is a hydrophilic polymer that is electrically neutralized as a whole. The non-ionic hydrophilic polymer is not particularly limited, but is preferably, for example, a homopolymer consisting of non-ionic hydrophilic monomers. Examples of the non-ionic hydrophilic polymer block include polyalkylene glycol (preferably polyethylene glycol (PEG)) and polyoxazoline (preferably poly(2-oxazoline)). In the above, the alkylene moiety may be, for example, $C_{2-6}$ alkylene or $C_{2-4}$ alkylene.

In a preferred embodiment, the non-ionic hydrophilic polymer block has a large molecular weight such that the ligand is not exposed (revealed) in a complex formed of one molecule of ligand-modified nucleic acid and two molecules of

polycation which preferably has a branch. In a preferred embodiment, for example, the non-ionic hydrophilic polymer block is a linear polymer (having no branch) or a branched polymer. The branched polymer has one or more branches, and the branch has at least two or more (preferably two) polymer chains. Each polymer chain may have a number-average molecular weight of, for example, but not particularly limited to, 10 kDa or more, 15 kDa or more, 20 kDa or more, 25 kDa or more, 30 kDa or more, or 35 kDa or more. Each polymer chain may have a number-average molecular weight of, for example, but not particularly limited to, 100 kDa or less, 90 kDa or less, 80 kDa or less, 70 kDa or less, 60 kDa or less, 60 kDa or less, or 50 kDa or less. In a preferred embodiment, Mw/Mn < 1.2, < 1.15 or < 1.1 {wherein Mw represents a weight-average molecular weight and Mn represents a number-average molecular weight}. Each polymer chain may have, for example, but not particularly limited to, 10 kDa to 100 kDa, 20 kDa to 90 kDa, 25 kDa to 70 kDa, 30 kDa to 60 kDa, or 30 kDa to 50 kDa. The larger the polymer chain, the more preferable it is from the viewpoint of nucleic acid protection. In a preferred embodiment, the non-ionic hydrophilic polymer block is PEG.

According to the present disclosure, the non-ionic hydrophilic polymer block in the present disclosure is bulky, so that it is usually not easy for three or more molecules of polycation to simultaneously bind to one molecule of nucleic acid in order to form steric hindrance.

According to the present disclosure, also, when two molecules of polycation are associated with one molecule of nucleic acid, it is considered that the ligand is buried in the non-ionic hydrophilic polymer block and does not have accessibility to the target. In one embodiment, the polycation binds to one molecule of nucleic acid at a molecular number ratio of 1:1, and then may associate with the nucleic acid by a two-step complex formation process of binding at 1:2, and in the solution, an assembly associated at a molecular number ratio of 1:1 (1:1 assembly) also exists, and in the 1:1 assembly, the ligand enhances accessibility to the target. The existence probability of the 1:1 assembly may be important for targeting of the nucleic acid, and the existence probability of an assembly associated at a molecular number ratio of 1:2 (1:2 assembly) may be important for blood retention (particularly stability) of the nucleic acid. In one embodiment, it is considered that the 1:1 assembly further associates with a polycation to form a 1:2 assembly, or the 1:2 assembly dissociates from one molecule of polycation to form a 1:1 assembly, and the state dynamically changes. It is considered that the non-associated nucleic acid (or naked nucleic acid) or the nucleic acid in the 1:1 assembly presents the ligand in an accessible manner and is taken up by the target cell.

The polycation block (or cationic polymer block) is, for example, a cationic natural amino acid and a cationic non-natural amino acid, for example, a cationic natural amino acid such as histidine, tryptophan, ornithine, arginine and lysine, and/or a polymer block having

$-NH-(CH_2)_p-NH-$ {wherein p is a natural number of 1 to 10};
$-NH-(CH_2NH)_q-$ {wherein q is a natural number of 1 to 5}; or
$-NH-((CH_2)_sNH)_r-$ {wherein s is a natural number of 1 to 5, and r is a natural number of 1 to 5}
as a side chain, for example, a polymer block of a cationic non-natural amino acid having said cationic side chain, for example, a polymer block of a cationic non-natural amino acid such as aspartic acid or glutamic acid having said cationic side chain.

In one embodiment of the present invention, the cationic polymer or cationic polymer block is a polymer block having

$-NH-(CH_2)_p-NH-$ {wherein p is a natural number of 1 to 10};
$-NH-(CH_2NH)_q-$ {wherein q is a natural number of 1 to 5}; or
$-NH-((CH_2)_sNH)_r-$ {wherein s is a natural number of 1 to 5, and r is a natural number of 1 to 5}
as a side chain. Here, preferred examples of the cationic natural amino acid include histidine, tryptophan, ornithine, arginine and lysine, more preferred examples include arginine, ornithine and lysine, still more preferred examples include ornithine and lysine, and even more preferred examples include lysine. In one embodiment of the present invention, the cationic polymer or cationic polymer portion can be polylysine or polyornithine. The ends of these side chains may be, for example, a protecting group or H.

The cationic polymer or cationic polymer block may contain a mixture of cationic amino acids and amino acids having a cationic side chain. That is, in one embodiment of the present disclosure, the cationic polymer or cationic polymer block is a polymer of monomer units containing cationic natural amino acids, cationic non-natural amino acids, or cationic natural amino acids and cationic non-natural amino acids. In one embodiment of the present invention, the bond between monomer units in the cationic polymer or cationic polymer block is a peptide bond. In a preferred embodiment of the present disclosure, the cationic non-natural amino acid is an amino acid having

$-NH-(CH_2)_p-NH-$ {wherein p is a natural number of 1 to 10};
$-NH-(CH_2NH)_q-$ {wherein q is a natural number of 1 to 5}; or
$-NH-((CH_2)_sNH)_r-$ {wherein s is a natural number of 1 to 5, and r is a natural number of 1 to 5}

as a side chain.

In one embodiment of the present disclosure, the cationic polymer or cationic polymer block can be a cationic polymer or cationic polymer block formed by polymerizing cationic natural amino acids and aspartic acid and glutamic acid modified with

$-NH-(CH_2)_p-NH-$ {wherein p is a natural number of 1 to 10};
$-NH-(CH_2NH)_q-$ {wherein q is a natural number of 1 to 5}; or
$-NH-((CH_2)_sNH)_r-$ {wherein s is a natural number of 1 to 5, and r is a natural number of 1 to 5}
in any order. In one embodiment of the present invention, 40%, 50%, 60%, 70%, 80%, 90%, 95%, 98% or 100% of the monomer units in the polymer have
$-NH-(CH_2)_p-NH-$ {wherein p is a natural number of 1 to 10};
$-NH-(CH_2NH)_q-$ {wherein q is a natural number of 1 to 5}; or
$-NH-((CH_2)_sNH)_r-$ {wherein s is a natural number of 1 to 5, and r is a natural number of 1 to 5}
as a side chain. The ends of these side chains may be, for example, a protecting group or H.

In one embodiment, the cationic polymer block may have a structure of the following formula (I):

[Chemical Formula 1]

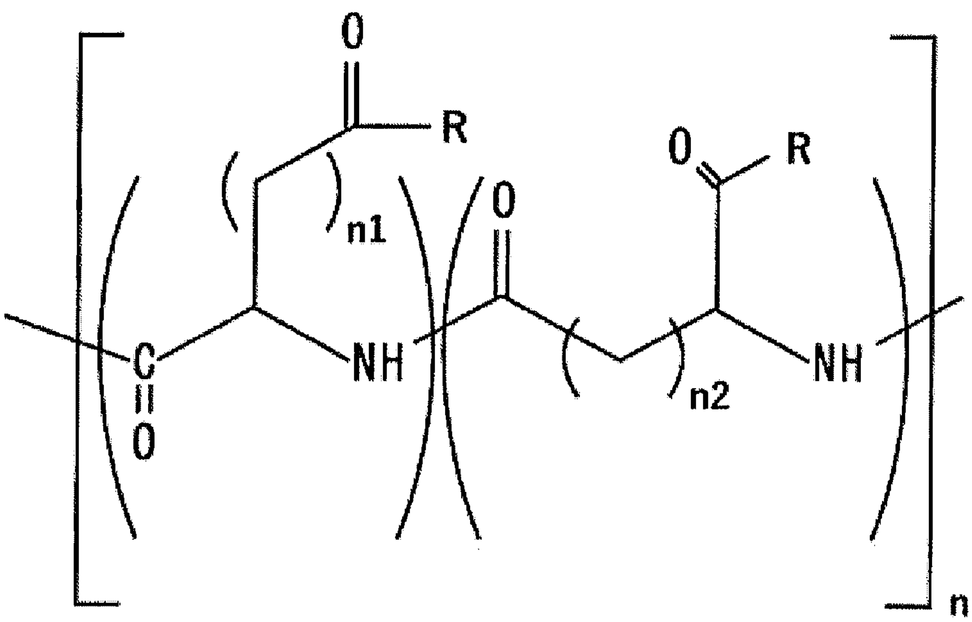
(I)

{wherein

R is
$-NH-(CH_2)_p-H$ {wherein p is a natural number of 1 to 10};
$-NH-(CH_2NH)_q-H$ {wherein q is a natural number of 1 to 5}; or
$-NH-((CH_2)_sNH)_r-H$ {wherein s is a natural number of 1 to 5, and r is a natural number of 1 to 5},
n is a natural number of 10 to 500 (can be 10 to 400, 10 to 300, 10 to 200, 10 to 80, 10 to 60, 10 to 40, 10 to 30 or 15 to 25), and n1 and n2 are 1 or 2}. Although not particularly limited, n1 and n2 may be the same or different, for example, both may be 1, or both may be 2. In the copolymer of the non-ionic hydrophilic polymer block and the cationic polymer block, one of the ends of the cationic polymer block may be linked to the non-ionic hydrophilic polymer block.

In a preferred embodiment, the polycation block can be a homopolymer, more preferably poly-L-lysine or poly-L-ornithine.

In a preferred embodiment, the number-average degree of polymerization of the polycation block can be 10 to 400, 10 to 300, 10 to 200, 10 to 80, 10 to 60, 10 to 40, 10 to 30 or 15 to 25. The number-average degree of polymerization of

the polycation block is not particularly limited, but can be, for example, equivalent to the number of bases for a double-stranded nucleic acid (for example, number of bases × 0.5 to 2.0, × 0.75 to 1.33, × 0.8 to 1.25, × 0.87 to 1.2, or × 0.91 to 1.1). In a preferred embodiment, the number-average degree of polymerization of the polycation block is not particularly limited, but can be, for example, about half the number of bases for a double-stranded nucleic acid in the case of a single-stranded nucleic acid (for example, number of bases × 0.25 to 1.0, × 0.38 to 0.67, × 0.4 to 0.62, × 0.43 to 0.6, or × 0.45 to 0.55). The ratio of the weight-average degree of polymerization to the number-average degree of polymerization can be, for example, 1 to 1.5, 1 to 1.4, 1 to 1.3, 1 to 1.2, or 1 to 1.1.

In the composition of the present disclosure, the charge ratio of the polycation to the nucleic acid (charge of polycation / charge of nucleic acid) is preferably 0.1 to 5. In the composition of the present disclosure, the charge ratio of the polycation to the nucleic acid (charge of polycation / charge of nucleic acid) is preferably 0.2 to 4, and can be 0.25 to 3, 0.3 to 2.5, or more preferably 0.5 to 2. In one embodiment, the charge ratio of the polycation to the nucleic acid (charge of polycation / charge of nucleic acid) is 1 or less, or less than 1. In one embodiment, the charge ratio is not about 1 (or 0.9 to 1.1). In one embodiment, the charge ratio can be 0.1 to 0.9, 0.2 to 0.8, 0.3 to 0.6, or 0.4 to 0.6, or about 0.5. In one embodiment, the charge ratio of one molecule of polycation to one molecule of nucleic acid is about 1 : about 1 (for example, 0.8:1.25 to 1.25:0.8), and the charge ratio of the polycation to the nucleic acid (charge of polycation / charge of nucleic acid) can be 0.1 to 0.9, 0.2 to 0.8, 0.3 to 0.6, or 0.4 to 0.6, or about 0.5.

In the composition of the present disclosure, the binding constant (Ka) between the polycation and the nucleic acid can be, for example, $3.0 \times 10^5$ ($M^{-1}s^{-1}$) or more.

In a preferred embodiment, the proportion of a complex consisting of one molecule of polycation and one molecule of nucleic acid in the complex consisting of the polycation and the nucleic acid in the composition of the present disclosure is 1% or more, 2% or more, 3% or more, 4% or more, 5% or more, 6% or more, 7% or more, 8% or more, 9% or more, 10% or more, 11% or more, 12% or more, 13% or more, 14% or more, 15% or more, 16% or more, 17% or more, 18% or more, 19% or more, 20% or more, 30% or more, 40% or more, 50% or more, 60% or more, 70% or more, 80% or more, or 90% or more. In a preferred embodiment, the proportion of a complex consisting of two molecules of polycation and one molecule of nucleic acid in the complex in the composition of the present disclosure is 90% or less, 80% or less, 70% or less, 60% or less, 50% or less, 40% or less, 30% or less, 20% or less, 19% or less, 18% or less, 17% or less, 16% or less, 15% or less, 14% or less, 13% or less, 12% or less, 11% or less, 10% or less, 9% or less, 8% or less, 7% or less, 6% or less, 5% or less, 4% or less, 3% or less, 2% or less, 1% or less. In a preferred embodiment, in the complex consisting of the polycation and the nucleic acid in the composition of the present disclosure, the proportion of a complex consisting of one molecule of polycation and one molecule of nucleic acid is 30% or more, and the proportion of a complex consisting of two molecules of polycation and one molecule of nucleic acid is 70% or less. In a preferred embodiment, in the complex consisting of the polycation and the nucleic acid in the composition of the present disclosure, the proportion of a complex consisting of one molecule of polycation and one molecule of nucleic acid is 40% or more, and the proportion of a complex consisting of two molecules of polycation and one molecule of nucleic acid is 60% or less. In a preferred embodiment, in the complex consisting of the polycation and the nucleic acid in the composition of the present disclosure, the proportion of a complex consisting of one molecule of polycation and one molecule of nucleic acid is 50% or more, and the proportion of a complex consisting of two molecules of polycation and one molecule of nucleic acid is 50% or less. In a preferred embodiment, in the complex consisting of the polycation and the nucleic acid in the composition of the present disclosure, the proportion of a complex consisting of one molecule of polycation and one molecule of nucleic acid is 60% or more, and the proportion of a complex consisting of two molecules of polycation and one molecule of nucleic acid is 40% or less. In a preferred embodiment, in the complex consisting of the polycation and the nucleic acid in the composition of the present disclosure, the proportion of a complex consisting of one molecule of polycation and one molecule of nucleic acid is 70% or more, and the proportion of a complex consisting of two molecules of polycation and one molecule of nucleic acid is 30% or less. In a preferred embodiment, in the complex consisting of the polycation and the nucleic acid in the composition of the present disclosure, the proportion of a complex consisting of one molecule of polycation and one molecule of nucleic acid is 80% or more, and the proportion of a complex consisting of two molecules of polycation and one molecule of nucleic acid is 20% or less. In a preferred embodiment, in the complex consisting of the polycation and the nucleic acid in the composition of the present disclosure, the proportion of a complex consisting of one molecule of polycation and one molecule of nucleic acid is 90% or more, and the proportion of a complex consisting of two molecules of polycation and one molecule of nucleic acid is 10% or less.

In the composition of the present disclosure, the nucleic acid can be a double-stranded nucleic acid or a single-stranded nucleic acid. In the composition of the present disclosure, the nucleic acid can be double-stranded DNA, single-stranded DNA, double-stranded RNA, or single-stranded RNA. In the composition of the present disclosure, the nucleic acid can include a modified nucleic acid. In the composition of the present disclosure, the nucleic acid may consist of a modified nucleic acid. In the composition of the present disclosure, the nucleic acid preferably includes double-stranded RNA. In the composition of the present disclosure, the nucleic acid is preferably double-stranded RNA. In the composition of the present disclosure, the nucleic acid is preferably siRNA or shRNA. In the composition of the present disclosure, the nucleic acid may be linked to a ligand that binds to a target expressed on the cell surface. Thereby, the nucleic acid binds to

a cell expressing the target, and uptake into the cell is promoted. The molecule targeted by the ligand is called a delivery target.

The nucleic acid can knockdown a target nucleic acid. The target of knockdown is called a knockdown target. Examples of the nucleic acid that is the knockdown target include, but are not particularly limited to, the following. Clusterin gene, Nucleolin gene, AKT1 protein kinase gene, BIRC5 gene, MAGEC1 gene, MAGEC2 gene, CTAG1 gene, TPBG gene, Hsp27 gene, beta-Catenin gene, CXCL12 (SDF-1) gene, STAT-3 gene, PKN3 gene, PLK1 gene, mutant KRAS (G12D) gene, Grb-2 gene, Androgen receptor gene, TGFβ gene (TGFbeta-1 gene, TGFbeta-2 gene, TGFbeta-3 gene), STAT-3 gene, VEGF gene, KSP (Eg5) gene, CEBPA gene, Nek2 gene, p53 gene, MUC1 gene, TPBG gene, HIF-1 alpha gene, RPN2 gene, EphA2 gene, RRM1 gene, CDC45 gene, six-1 gene, IGF-1 receptor gene, HoxA1 gene, IGFBP-2 gene, IGFBP-5 gene, EGF receptor gene, Raf-1 gene, mTOR gene, Bcl-2 gene, Casein kinase-2 gene, KRAS gene, c-Myc gene, COX-2 gene, beta-3 tubin gene, ITCH gene, VEGF gene, VEGF receptor 2 gene, SIP1 gene, AGT gene, ERV-9 LTR gene, EVI1 gene, TNF-α gene, PAX-2 gene, Srpx2 gene, IRS-1 gene, Survivin gene, DUSP6 gene, HPV E6/E7 gene, HSPA9 gene, mitochondrial RNA (non-coding mitochondrial RNA), EWS FLI1 gene, SRC-3 gene, MDR1 gene, NTRK1 gene, NTRK2 gene, MDX3 gene, NR2F6 gene, MYD88 gene, NOTCH1 gene, β-3 integrin gene, c-FLIP gene, MADD gene, HER2 gene, CCAT2 gene, CTCFL gene, HIF-2α gene, BMI-1 gene, NETO-2 gene, CTFR gene, PD-1 gene, PD-L1 gene, PD-L2 (B7-DC (CD273)) gene, CLTA4 gene, HLA gene (HLA-A, HLA-B, HLA-C, HLA-DR, HLA-DP, HLA-DQ, HLA-E, HLA-G), MCH gene, 4-1BB gene, 4-1 BBL gene, CD3 gene (CD3α, CD3β, CD3γ, CD3δ, CD3ε, CD3ζ gene, IL-6 gene, IL-17 gene, IL-23 gene, ICOS gene, CD70 gene, CD27 gene, OX40 gene, OX40L gene, TL1A gene, DR3 gene, GITRL gene, GITR gene, CD30L gene, CD30 gene, TIM1 gene, TIM1L gene, TIM4 gene, SLAM gene, CD48 gene, CD58 gene, CD2 gene, CD155 gene, CD112 gene, CD226 gene, CD80 (B7-1) gene, CD86 (B7-2) gene, B7-H2 gene, LIGHT gene, HVEM gene, CD40 gene, CD40L gene, Galectin 9 gene, CD113, Collagen gene, CD160 gene, LAG3 gene, PSA gene, PSMA gene, PSCA gene, STEAP gene, BIRC5 gene, MAGEC1 gene, MAGEC2 gene, CTAG1 gene, TPBG gene, or molecules such as proteins encoded by these genes, or mRNA encoding proteins encoded by these genes, proteins known as the HDGF family (prototype protein, HDGF; HRP-1, HRP-2, HRP-3, HRP-4 (HRP = HDGF Related Protein)); and LEDGF (particularly, HRP-3)), TrkB receptor, or genes or mRNA encoding these receptors, miRNA such as miR-34, miR-34a, miR-16, miR-155, miR-17, miR-17-92, miR-215, let-7, miR-34, miR-10b, miR-3157, miR-34, miR-7, miR-21, miR-574-5p, miR-221, miR-484, miR-205, miR-210, miR-3189-3p, miR-3151, miR-199, miR-101, miR-96, miR-182.

HSP47 gene, α1-antitrypsin gene, ALAS-1 gene, DGAT2 gene, Hydroxyacidoxidase gene, TGFβ gene, TGFβ-2 gene, Transthyretin gene, PCSK9 gene, or molecules such as proteins encoded by these genes, or mRNA encoding proteins encoded by these genes, miR-103, miR-107.

The nucleic acid is linked to the ligand directly or indirectly via a spacer by a covalent bond. The ligand is a molecule capable of binding to a target molecule. The ligand can be, for example, a sugar (e.g., glucose). Sugar can bind to GLUT1 expressed on brain vascular endothelial cells, and therefore can target the brain. The ligand can also be, for example, a sugar chain (e.g., N-acetylgalactosamine (GalNAc)). The ligand can also be, for example, a peptide, such as an RGD peptide or a cyclic RGD peptide. The RGD peptide includes a tripeptide of arginine-glycine-aspartic acid and has cell adhesion actilvity. The RGD peptide can be, for example, 3 to 14 amino acids long, for example, 4 to 10 amino acids long. The RGD peptide can be, for example, a fragment of a natural protein (e.g., fibronectin, vitronectin, osteopontin, syndecan, laminin, etc.). The RGD peptide can be, for example, GRGDS (SEQ ID NO: 5). The RGD peptide may be cyclic. The cyclic RGD peptide (cRGD) peptide can be cyclo-RGDfK (SEQ ID NO: 7), cyclo-RGDyV (SEQ ID NO: 8), cyclo-RGDfY (SEQ ID NO: 9), cyclo-RGDyK (SEQ ID NO: 10), with cyclopentapeptide (c-RGFfV) (SEQ ID NO: 6) and c-RGDfV as lead compounds. Here, cyclo means cyclic, and lowercase letters mean D amino acids. The ligand is also preferably, for example, a peptide, for example, a sequence of LDV sequence, REDV sequence (SEQ ID NO: 11), YIGSR sequence (SEQ ID NO: 12), PDSGR sequence (SEQ ID NO: 13), RYVVLPR sequence (SEQ ID NO: 14), LGTIPG sequence (SEQ ID NO: 15), RNIAEIIKDI sequence (SEQ ID NO: 16), IKVAV sequence (SEQ ID NO: 17), LRE sequence, DGEA sequence (SEQ ID NO: 18), and HAV sequence, more preferably RGD sequence, YIGSR sequence (SEQ ID NO: 19), PDSGR sequence (SEQ ID NO: 20)2, LGTIPG sequence (SEQ ID NO: 21), IKVAV sequence (SEQ ID NO: 22) and HAV sequence, and particularly preferably RGD sequence. Among the RGD sequences, ERGD sequence (SEQ ID NO: 23) may be preferable. The ligand can also be a GLUT1 ligand. In the present specification, "GLUT1 ligand" means a substance that specifically binds to GLUT1. Various ligands are known as GLUT1 ligands, and examples thereof include, but are not particularly limited to, molecules such as glucose and hexose, and any GL3UT1 ligand can be used for preparing a carrier or conjugate in the present disclosure instead of glucose. The GLUT1 ligand preferably has an affinity for GLUT1 equal to or higher than that of glucose. 2-N-4-(1-azi-2,2,2-trifluoroethyl)benzoyl-1,3-bis(D-mannose-4-yloxy)-2-propylamine (ATB-BMPA), 6-(N-(7-nitrobenz-2-oxa-1,3-diazol-4-yl)amino)-2-deoxyglucose (6-NBDG), 4,6-O-ethylidene-alpha-D-glucose, 2-deoxy-D-glucose and 3-O-methylglucose are also known to bind to GLUT1, and these molecules can also be used as GLUT1 ligands in the present disclosure.

The ligand can be linked to the 5' end or 3' end of the nucleic acid. The ligand can also be linked to the 5' end or 3' end of the sense strand of the double-stranded nucleic acid, or the 5' end or 3' end of the antisense strand. The ligand can be particularly linked to the 5' end or 3' end of the sense strand. In a preferred embodiment, the ligand can be linked to the

backbone of the nucleic acid via a bond such as a phosphodiester bond, a phosphorothioate bond, or a peptide bond with the terminal nucleic acid.

In the composition of the present disclosure, preferably, in the complex of the polycation and the nucleic acid, the polycation encapsulates the nucleic acid linked to the ligand. Thereby, the blood retention of the nucleic acid is improved. In one embodiment, the blood retention of the nucleic acid is improved as compared with a naked nucleic acid. A naked nucleic acid refers to a nucleic acid that is not interacting with other macromolecules in a free state. On the other hand, in the composition of the present disclosure, the accessibility of the ligand to external molecules can be maintained at least partially. Therefore, migration ability to the target is maintained at least partially by the ligand. Therefore, in the composition of the present disclosure, the nucleic acid has improved migration ability toward the target as compared with a complex containing a nucleic acid not linked to a ligand.

According to the present disclosure, in the complex of the polycation and the nucleic acid, it is considered that one molecule or two molecules of polycation are associated with one molecule of nucleic acid.

The spacer is not particularly limited but may be stable in vivo. The spacer may contain a site cleavable under a reducing environment or an endosomal pH environment. The spacer may have a non-ionic hydrophilic polymer block.

In one embodiment, the subject has a disease or a condition requiring treatment or therapy in a specific tissue, and in the uPIC, the nucleic acid is linked to a ligand targeting the tissue, and the nucleic acid is delivered to the tissue to exert a preventive effect or a therapeutic effect.

The nucleic acid may link a compound of formula (Im1) or formula (Im2).

In one embodiment, the ligand is GalNAc, and the composition is used to migrate the nucleic acid to liver parenchymal cells. Migration of the nucleic acid to the liver can be beneficial, for example, for knockdown of a target gene in the liver, and prevention and/or treatment of liver disease thereby. In one embodiment, the ligand is an RGD peptide or a cRGD peptide, and the composition is used to migrate the nucleic acid to stellate cells of the liver (hepatic stellate cells). Migration of the nucleic acid to hepatic stellate cells can be beneficial, for example, for knockdown of a gene in hepatic stellate cells, and prevention and/or treatment of liver fibrosis thereby. In addition, prevention and treatment of liver fibrosis can be prevention of liver cirrhosis.

In one embodiment, the non-ionic hydrophilic polymer block is linked to the polycation at one end, but may not have a ligand at the other end. In a preferred embodiment, the polycation is not linked to a ligand.

The composition of the present disclosure may further contain pharmaceutically acceptable additives in addition to the complex. Examples of the additive include, but are not particularly limited to, pH adjusters, salts, isotonic agents, physiological saline (or water), and surfactants. The composition of the present disclosure may be formulated for parenteral administration (not particularly limited, for example, for intravenous administration or for hepatic arterial injection).

<Administration Method of the Present Disclosure>

According to the present disclosure, a method for administering a nucleic acid in a subject is provided. The subject may be a subject in need of administration of the nucleic acid.

The subject may have a disease or a condition requiring medical treatment with the present nucleic acid. According to the present disclosure, the nucleic acid can link the above ligand. The subject may also have one or more diseases or conditions requiring treatment selected from the group consisting of nervous systems such as brain and nerves; sensory organs such as eyes, ears, nose, and tongue; digestive organs such as esophagus, stomach, duodenum, small intestine, and large intestine; gallbladder, pancreas, liver, etc.; urinary organs such as kidney, ureter, and bladder; respiratory organs such as lungs, trachea, and bronchi; skeleton such as bones and muscles; skin; blood, lymph, spleen, thymus, lymph nodes, bone marrow, etc. To such a subject, a nucleic acid linked to a ligand targeting the tissue or organ having the disease or condition, or a complex containing the nucleic acid can be administered. In one embodiment, the subject may have, for example, a brain disease, the subject may have, for example, cancer, the subject may have, for example, a liver disease, wherein a nucleic acid linked to a ligand targeting the brain, a ligand targeting cancer, and a ligand targeting the liver is administered to each subject, respectively. According to the present disclosure, the nucleic acid can be administered to the subject in the form of the above complex with the polycation. Specifically, the nucleic acid can be administered as the composition of the present disclosure. In addition, the nucleic acid can be used in combination with the above polycation. Administration can be parenteral administration, for example, intravenous administration. In addition, when it is desired to effectively migrate the nucleic acid to the liver, the nucleic acid may be administered by hepatic arterial injection.

In one embodiment, the ligand is GalNAc, and the subject may benefit from gene expression or knockdown of a target gene in the liver, and prevention and/or treatment of liver disease thereby. In one embodiment, the ligand is an RGD peptide or a cRGD peptide, and the subject may benefit from gene expression or knockdown of a gene in hepatic stellate cells, and prevention and/or treatment of a liver disease or condition (e.g., liver fibrosis) thereby.

According to the present disclosure, a polycation or a composition containing the polycation, for use in the

method of the present disclosure is provided. According to the present disclosure, also, a nucleic acid or a composition containing the nucleic acid for use in the method of the present disclosure is provided. According to the present disclosure, also, a complex or a composition containing the complex for use in the method of the present disclosure is provided. According to the present disclosure, the nucleic acid can link the above ligand.

According to the present disclosure, use of a polycation in the manufacture of a composition for use in the method of the present disclosure is provided. According to the present disclosure, also, use of a nucleic acid in the manufacture of a composition for use in the method of the present disclosure is provided. According to the present disclosure, also, use of a complex in the manufacture of a composition for use in the method of the present disclosure is provided. According to the present disclosure, the nucleic acid can link the above ligand.

EXAMPLES

(1) Materials

Two-branched poly(ethylene glycol)-b-poly(L-ornithine) (hereinafter referred to as "2-arm PEG-PLO") was purchased from NOF Corporation (Tokyo, Japan). The number-average molecular weight of the PEG chain was 2 × 40 kDa, and the number-average degree of polymerization of PLO was 20. Sterile HEPES buffer (1 M, pH 7.3) was purchased from Amresco (Solon, OH, USA). Biophen(TM) FVII kit was purchased from Hyphen BioMed (Neuville-sur-Oise, France). siRNA molecules encoding human polo-like kinase 1 (siPLK1) and ligand-conjugated siPLK1 were synthesized by GeneDesign, Inc. (Osaka, Japan).

The sequence of siPLK1 was as follows:

5'-G(M)^A(M)^A(M)G(M)A(M)U(M)C(F)A(M)C(F)C(F)C(F)U(M)C(M)C(M)U(M)U(M)A(M)A(M)A(M)U(M)A(M)-3' (sense strand of siPLK1; SEQ ID NO: 1) and

5'-U(M)^A(F)^U(M)U(M)U(M)A(F)A(M)G(M)G(M)A(M)G(M)G(M)G(M)U(F)G(M)A(F)U(M)C(M)U(M)U(M)C(M)^U(M)^U(M)-3' (antisense strand of siPLK1; SEQ ID NO: 2).

Here, capital letters indicate RNA. In addition, M shown in parentheses indicates 2'-OMe modification {wherein "Me" represents methyl} of the base shown immediately before, and F indicates 2'-F modification of the base shown immediately before, respectively. The symbol "^" indicates phosphorothioate (PS) modification.

siRNA against Factor VII linked with GalNAc (siFVII) was synthesized. The siRNA had the following sequences:

5'-C(M)^A(M)^G(M)G(M)A(M)U(M)C(F)A(M)U(F)C(F)U(F)C(M)A(M)A(M)G(M)U(M)C(M)U(M)U(M)A(M)C(M)-3' (SEQ ID NO: 3) and

5'-G(M)^U(F)^A(M)A(M)G(M)A(F)C(M)U(M)U(M)G(M)A(M)G(M)A(M)U(F)G(M)A(F)U(M)C(M)C(M)U(M)G(M)^U(M)^U(M)-3' (SEQ ID NO: 4).

Here, capital letters indicate RNA. In addition, M shown in parentheses indicates 2'-OMe modification {wherein "Me" represents methyl} of the base shown immediately before, and F indicates 2'-F modification of the base shown immediately before, respectively. The symbol "^" indicates phosphorothioate (PS) modification.

Alexa647-siPLK1 had Alexa Fluor (trademark) 647 dye added to the 5' end of the sense strand. Tri-N-acetylgalactosamine-PEG9-dibenzocyclooctyne (GalNAc-DBCO) was introduced to the 3' end of the sense strand using azide PEG4 NHS ester and amino C6 linker (see formula (Im1)). Cyclo(Arg-Gly-Asp-D-Phe-Lys) having a cysteine unit was conjugated to the 3'-end of the sense strand using N-(6-maleimidocaproyloxy)succinimide (cRGD-EMCS) and C6 linker (see formula (Im2)). The ligand-conjugated Alexa647-siRNA has Alexa647 bound to the 3' end of the antisense strand.

[Chemical Formula 2]

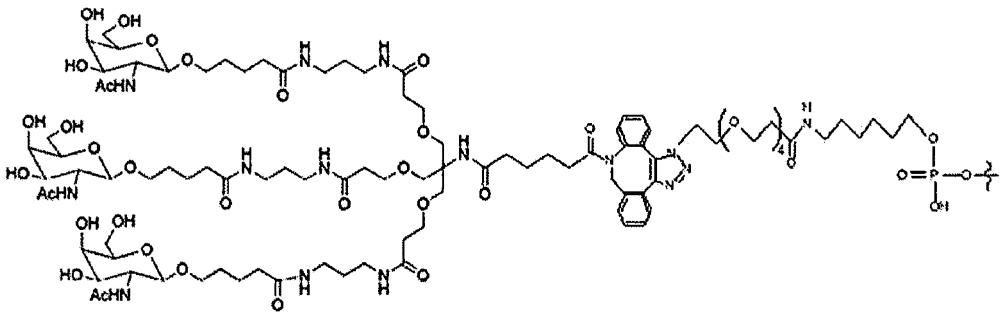

(I m 1)

[Chemical Formula 3]

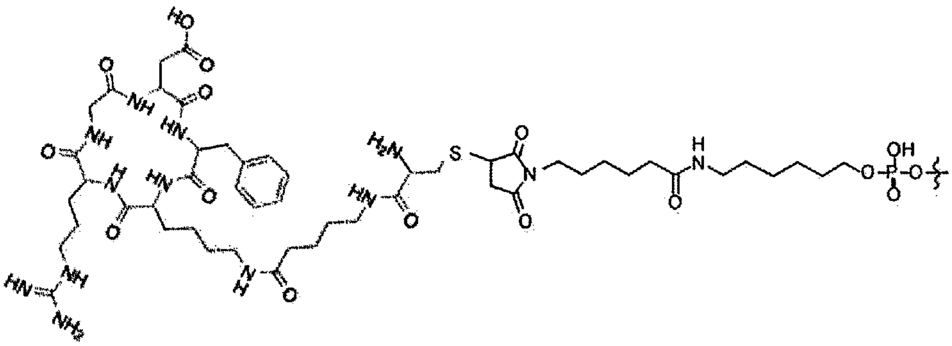

(I m 2)

(2) Animal Experiments

All animal experiments have been approved by the Ethics Committee of the Innovation Center of NanoMedicine (Kawasaki, Japan). Balb/c and Balb/c nu/nu mice (6-8 weeks old female, weight 18-20 g) were purchased from Charles River Laboratories Japan (Kanagawa, Japan).

(3) Preparation of Unit Polyion Complex (uPIC)

siRNA and polymer were separately dissolved in 10 mM HEPES buffer (pH 7.3), and PIC was formed by changing the molar ratio (N/P ratio) of primary amine (N) of Orn residue in the polymer and phosphate (P) of siRNA, and mixed.

Hydrodynamic diameters of siPLK1, GalNAc-modified siPLK1, and uPIC were measured by fluorescence correlation spectroscopy (FCS). FCS measurements (repetition number 10) were performed using LSM880 (Carl Zeiss, Oberkochen, Germany) equipped with a 40x water immersion objective lens and a He-Ne laser (633 nm). Alexa647-siRNA solution was mixed with the polymer solution such that the molar ratio (A/P) of primary amine of the polycationic segment and phosphate of siRNA was 1 (final concentration = 10 μM). This uPIC sample was diluted with 10 mM HEPES buffer (pH 7.3) containing 150 mM NaCl to a concentration of 10 nM siRNA. The hydrodynamic diameter (DH) was calculated from the following Stokes-Einstein equation using the diffusion coefficient (DC) of each PIC sample based on the DC of Cy5 dye.

DH = kBT / 3$\pi\eta$DC {wherein kB is the Boltzmann constant, T is the temperature, and $\eta$ is the solvent viscosity.}

In FCS, the number of fluorescent particles (N) can be calculated from the magnitude of fluctuation of fluorescence intensity in the focal region (Rigler, R.; Elson, E. S. Fluorescence Correlation Spectroscopy: Theory and Applications; Springer: Berlin, 2001). Since the fluorescence measured in this observation is derived from siRNA, the association number of siRNA in the fluorescent particle (ANRNA) can be calculated by the following formula.

ANRNA = NRNA / NPIC {wherein NRNA is the number of fluorescent particles in the naked siRNA solution containing no polymer, and NPIC is the number of fluorescent particles in the PIC solution.}

(4) Intravital Confocal Microscopy Observation

Intraocular observation in this study was performed using a Nikon A1R confocal laser scanning microscope (Nikon Corporation, Tokyo, Japan) equipped with an upright ECLIPSE FN1. The obtained data was analyzed using NIS-Elements software to quantify fluorescence intensity. A 40x objective lens was used for observation of liver and kidney, and a 20x objective lens was used for observation of earlobe.

The blood circulation profile of uPIC (1.8 nmol siRNA/mouse) prepared with Alexa647-labeled siRNA was measured using a 640 nm diode laser and a 700/75 nm bandpass emission filter (Nikon Corporation, Tokyo, Japan). The fluorescence intensity derived from Alexa647 in the earlobe vein was quantified, and the relative fluorescence intensity was calculated by the following formula:

Relative fluorescence intensity (%) = (Intensity at indicated time point - Minimum intensity) / (Maximum intensity - Minimum intensity) * 100.

For liver imaging, eFluor(TM) 450-CD31 antibody (Thermo Fisher, Massachusetts, USA) was used to visualize the sinusoidal wall. Alexa Fluor(TM) 488-$\alpha$SMA antibody (Abcam, Cambridge, UK) was used as a marker for hepatic stellate cells. Each fluorescently labeled antibody and Alexa647-siRNA were excited with 405, 488, and 640 nm lasers and detected with 450/50, 525/50, and 700/75 bandpass emission filters, respectively. Each antibody was injected intravenously at a dose of 10 $\mu$g/mouse. Measurement was started 30 minutes after injection, and uPIC was administered 1 minute later. After measurement, multiple regions of interest (ROI) were taken in liver parenchymal cells, the background before uPIC administration was subtracted from the graph of the time course of fluorescence intensity, and the area under the curve (AUC) from 0 to 6 hours was calculated.

(5) Gene Silencing Test of GalNAc-uPIC

The prepared uPIC (1.8 nmol siRNA/mouse) was injected into the tail vein of anesthetized female 8-week-old Balb/c mice. Seven days after administration, blood was collected from the tail vein of anesthetized mice, and the amount of FVII protein was measured using Biophen FVII kit according to the manufacturer's protocol. The amount of FVII protein in physiological saline-administered mice was normalized to 1.

(6) Gene Silencing Test of cRGD-uPIC

BxPC3 cells (1 $\times$ $10^7$ cells/mouse/100 microL) were subcutaneously inoculated into the right flank of BALB/c nude female mice (8 weeks old). Tumors were allowed to grow to ~100 $mm^3$ before intravenous injection of uPICs (1.8 nmol siRNA/mouse). Tumors were excised 48 hours after injection and homogenized using Multi-Beads Shocker (Yasui Kikou, Osaka, Japan). RNA was collected using RNeasy Mini kit (QIAGEN, Valencia, CA, USA) according to the manufacturer's instructions. After adjusting the RNA concentration, genomic DNA was removed, and then cDNA synthesis was performed using ReverTra Ace(TM) Master Mix (Toyobo, Tokyo, Japan). Real-time reverse transcription polymerase chain reaction (rt-PCR) was performed using ABI 7500 Fast Real-time rt-PCR System and TaqMan(TM) Fast Universal PCR Master Mix (Thermo Fisher Scientific, Waltham, MA, USA). Actin was used as a housekeeping gene, and the obtained data was normalized before statistical analysis.

Results

It is considered that the polycation having a branched non-ionic hydrophilic polymer forms the complex (uPIC) shown in FIG. 1 in an aqueous solution with siRNA. Here, the complex can contain one molecule of siRNA and two molecules of polycation, and the polycation associates with the anionic siRNA by electrostatic interaction. The hydrodynamic diameter of siRNA measured using FCS is about 4 nm, and the hydrodynamic diameter of GalNAc-modified siRNA is about 5 nm. On the other hand, the hydrodynamic diameter of uPIC is about 21 nm. Considering this, in the

obtained uPIC, the bulky non-ionic hydrophilic polymer wraps the siRNA, which is considered to protect the siRNA from degradation and elimination. According to scaling theory, the hydrodynamic diameter (DH) of PEG can be calculated from the following formula.

$$DH = 0.029 \times (\text{Molecular weight of PEG})^{0.571 \pm 0.009}$$

(Literature: K. Devanand and J. C. Selser, Macromolecules 1991, 24, 5943-5947)

Considering that the hydrodynamic diameter is 5 to 6 nm when PEG is 10 kDa, when the number-average molecular weight of PEG is 10 kDa or more, siRNA and the targeting agent labeled on siRNA are buried in PEG and are considered to be protected from degradation and elimination. In fact, when the blood retention of uPIC was evaluated, the blood retention was remarkably improved when the NP ratio (N/P) was improved (see FIG. 2). On the other hand, GalNAc-linked siRNA generally had reduced blood retention (see FIG. 2). This result indicates that linking GalNAc to siRNA (see formula (Im1)) changed the pharmacokinetics of uPIC.

According to FIG. 2, no significant difference was observed in blood retention between the case where the NP ratio was 1 and the case where it was 2. When the NP ratio is 1 or more, the possibility that the nucleic acid and the polycation are associated at a molecular number ratio of 1:2 is relatively high. When the NP ratio is 0.5, it is considered that the possibility that the nucleic acid and the polycation are associated at a molecular number ratio of 1:1 is relatively high, but it is considered that the presence of the bulky non-ionic hydrophilic polymer block contributed to the improvement of nucleic acid stability by one molecule of cationic polymer even in the 1:1 assembly. In the uPIC in which the nucleic acid and the polycation are associated at a molecular number ratio of 1:2, the targeting molecule was considered to be buried in the complex by the bulky non-ionic hydrophilic polymer block and not exposed on the surface of the complex, but the uPIC in which the nucleic acid and the polycation are associated at a molecular number ratio of 1:1 is considered to expose the targeting molecule on the surface of the uPIC while stabilizing the nucleic acid as described above. Furthermore, since the polycation has the property of adsorbing to sinusoidal endothelium over time, when the NP ratio is 1 or more, it is considered that the possibility that the nucleic acid and the polycation are initially associated at a molecular number ratio of 1:2 is relatively high, but it was predicted that the proportion of the complex in which the nucleic acid and the polycation are associated at a molecular number ratio of 1:1 increases over time.

The accumulation of uPIC containing GalNAc-linked siRNA in the liver was evaluated. The results showed that the accumulation of uPIC in the liver increased by linking GalNAc to siRNA as shown in FIG. 3A. This is considered to be due to the targeting effect by GalNAc. When the accumulation of siRNA in liver parenchymal cells was quantified, it was revealed that linking GalNAc showed an accumulation in the liver of 10 times or more compared to uPIC containing GalNAc-unlinked siRNA as shown in FIG. 3B.

GalNAc-linked siFVII (1.8 nmol/animal) was administered from the tail vein of mice (balb/c), blood was collected 7 days later, and FVII in blood was quantified with Biophen FVII kit. The value of the physiological saline administration group was normalized as 1. The polymer dose was 0.125, 0.25, 0.5, 1.25 mg/animal for N/P = 0.5, 1, 2, 5, respectively. The results were as shown in FIG. 4.

As shown in FIG. 4, GalNAc-unlinked uPIC did not show a significant knockdown effect on FVII, but GalNAc-linked uPIC showed a significant knockdown effect on FVII. The knockdown effect appeared strongly when the NP ratio was 0.5 to 2, and particularly strongly when the NP ratio was 0.5 to 1. As described above, in uPIC containing nucleic acid:cationic polymer at 1:2, the nucleic acid and its modifying group are covered with the bulky non-ionic hydrophilic polymer possessed by the cationic polymer, and it is considered difficult to target cells by the modifying group. When the NP ratio is 2 or more, the proportion of uPIC containing nucleic acid:cationic polymer at 1:2 increases, so it is considered possible that only a weak silencing effect was exhibited compared to uPIC formed with a low NP ratio. Also, it is expected that the nucleic acid and cationic polymer taken up into liver parenchymal cells form uPIC. However, since uPIC does not have endosomal escape ability, it is considered to be excreted in bile as it is. Therefore, from the viewpoint of enhancing the silencing effect after cell uptake, it was suggested that a smaller amount of cationic polymer might be preferable. In addition, the fact that improved blood retention and high silencing efficiency were shown at an NP ratio of 0.5 suggests that the nucleic acid repeatedly associates with and dissociates from the polycation, improving blood retention by temporary association and improving targeting by temporary dissociation.

Linking another ligand to siRNA was attempted. Here, cRGD peptide was used as the ligand. A uPIC containing cRGD-linked siRNA (see formula (Im2)) was prepared, and blood retention was evaluated. As shown in FIG. 5, the results suggested that blood retention improves as the NP ratio increases. Also, as shown in FIG. 5, no effect on blood retention due to the linkage of cRGD was observed.

Migration of uPIC containing cRGD-linked siRNA to the liver was confirmed by an in vivo test. The results were as shown in FIG. 6, and the uPIC containing cRGD-linked siRNA showed localization different from that of uPIC containing ligand-free (unlinked) siRNA, and also showed localization different from that of uPIC containing GalNAc-linked siRNA.

Localization of Alexa488-labeled anti-α smooth muscle actin (αSMA) antibody and uPIC was examined. As a result, uPIC showed co-localization with anti-αSMA antibody as shown in FIG. 7. This indicates that uPIC accumulates in stellate cells in the liver.

In uPIC, the internal siRNA is confined inside by steric hindrance due to the bulky non-ionic hydrophilic polymer, and a protective barrier against biochemical degradation of siRNA is formed, improving the blood retention of siRNA. From this, it is considered that access to the outside is also restricted for the siRNA itself. In this example, an unexpected result was obtained indicating that labeling on siRNA changes the pharmacokinetics of siRNA. This effect was remarkable when the NP ratio in uPIC was approximately 5 or less. This result suggests that the labeling on siRNA is not completely closed to access from the outside when the NP ratio is approximately 5 or less. The results of this example show the possibility of a drug delivery system by labeling of siRNA in uPIC.

Content of Sequence Listing

Sequence Number (ID): 1 gaagatcacc ctccttaaat a 21
Sequence Number (ID): 2 tatttaagga gggtgatctt ctt 23
Sequence Number (ID): 3 caggatcatc tcaagtctta c 21
Sequence Number (ID): 4 gtaagacttg agatgatcct gtt 23
Sequence Number (ID): 5 GRGDS 5
Sequence Number (ID): 6 RGFXV 5
Sequence Number (ID): 7 RGDXK 5
Sequence Number (ID): 8 RGDXV 5
Sequence Number (ID): 9 RGDXY 5
Sequence Number (ID): 10 RGDXK 5
Sequence Number (ID): 11 REDV 4
Sequence Number (ID): 12 YIGSR 5
Sequence Number (ID): 13 PDSGR 5
Sequence Number (ID): 14 RYVVLPR 7
Sequence Number (ID): 15 LGTIPG 6
Sequence Number (ID): 16 RNIAEIIKDI 10
Sequence Number (ID): 17 IKVAV 5
Sequence Number (ID): 18 DGEA 4
Sequence Number (ID): 19 YIGSR 5
Sequence Number (ID): 20 PDSGR 5
Sequence Number (ID): 21 LGTIPG 6
Sequence Number (ID): 22 IKVAV 5
Sequence Number (ID): 23 ERGD 4

## Claims

**1.** A composition comprising a complex of a polycation and a nucleic acid,

wherein the nucleic acid is covalently linked to a ligand for a target,
the polycation is in the form of a block copolymer of a branched non-ionic hydrophilic polymer block and a polycation block,
the branched non-ionic hydrophilic polymer comprises at least one branch and at least two non-ionic hydrophilic polymers extending from the said branch, and
relative to the absence of the polycation, the composition improves blood retention of the nucleic acid, and, relative to a complex comprising a nucleic acid not linked to the ligand, improves migration ability toward the target.

**2.** The composition according to claim 1, wherein a charge ratio (charge of polycation / charge of nucleic acid) of the polycation to the nucleic acid contained in the composition is 0.1 to 5.

**3.** The composition according to claim 1 or 2, wherein each non-ionic hydrophilic polymer has a number-average molecular weight of 10 kDa or more.

4. The composition according to any one of claims 1 to 3, wherein each non-ionic hydrophilic polymer is a polyalkylene glycol.

5. The composition according to any one of claims 1 to 4, wherein the polycation block has a number-average degree of polymerization of 15 to 30.

6. The composition according to any one of claims 1 to 5, wherein the polycation block is poly-L-lysine or poly-L-ornithine.

7. The composition according to any one of claims 1 to 6, wherein the ligand is a sugar or a polysaccharide.

8. The composition according to any one of claims 1 to 7, wherein the polycation is a block copolymer of a branched non-ionic hydrophilic polymer that comprises polyethylene glycol (PEG) each having a number-average molecular weight of 10 kDa or more, and a polycation block comprising poly-L-lysine or poly-L-ornithine, and wherein the polycation block has a number-average degree of polymerization of 15 to 30.

9. The composition according to any one of claims 1 to 8, wherein the ligand is an RGD peptide or a cRGD peptide.

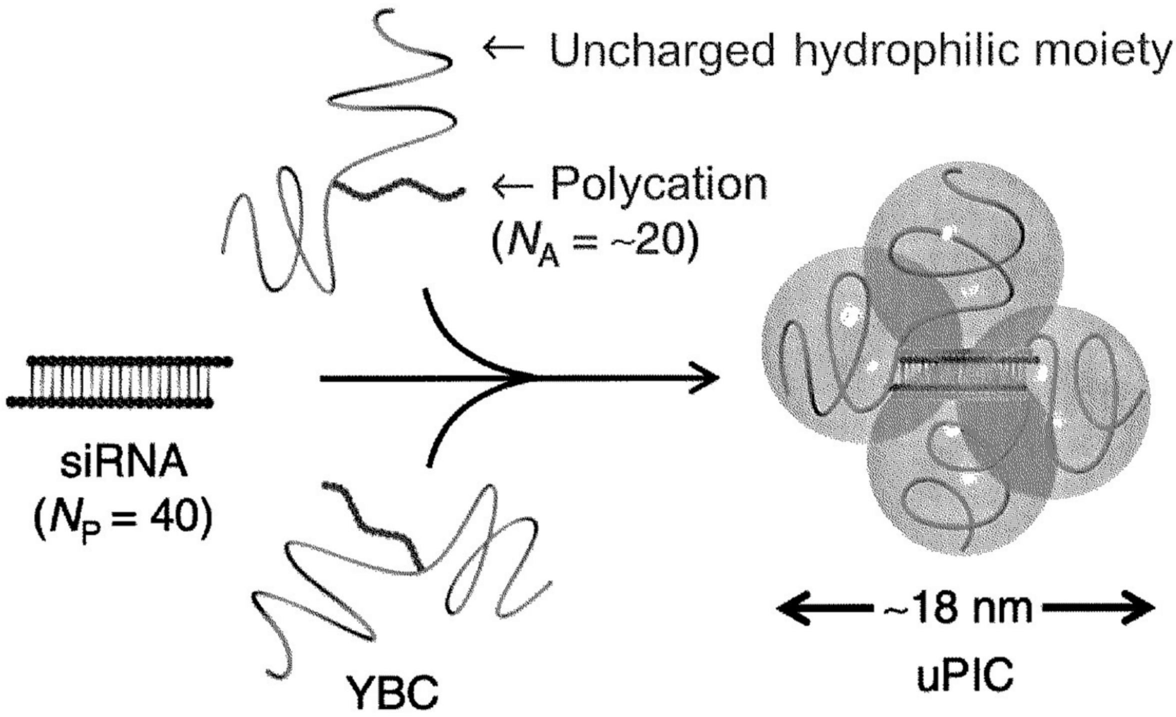
← Uncharged hydrophilic moiety
← Polycation
($N_A$ = ~20)
siRNA
($N_P$ = 40)
YBC
~18 nm
uPIC

FIG. 1

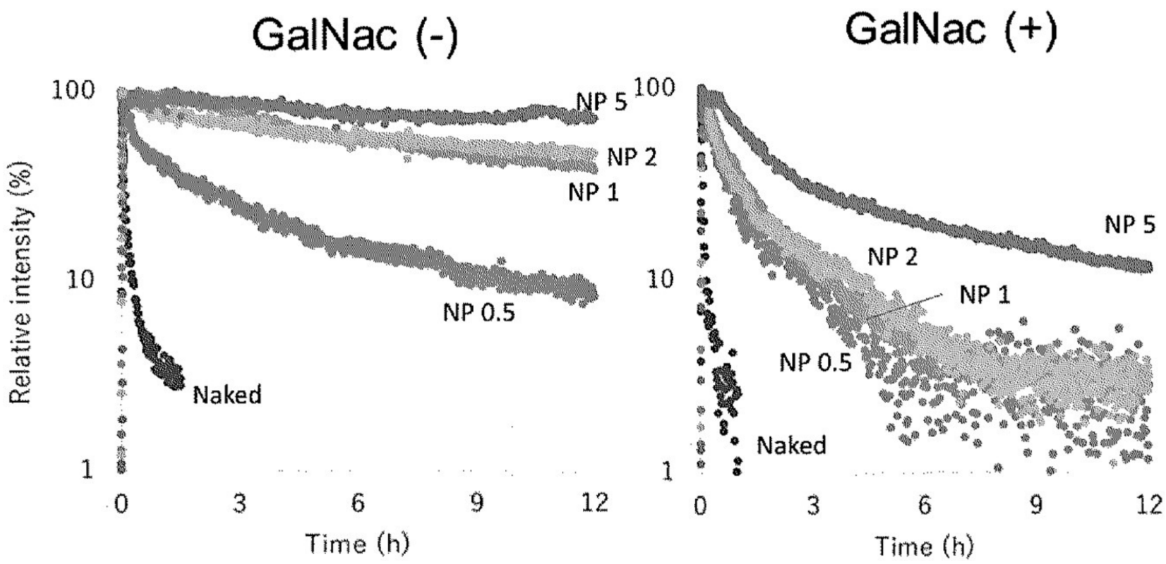
GalNac (-)
GalNac (+)
Relative intensity (%)
100
10
1
0
3
6
9
12
Time (h)
NP 5
NP 2
NP 1
NP 0.5
Naked

FIG. 2

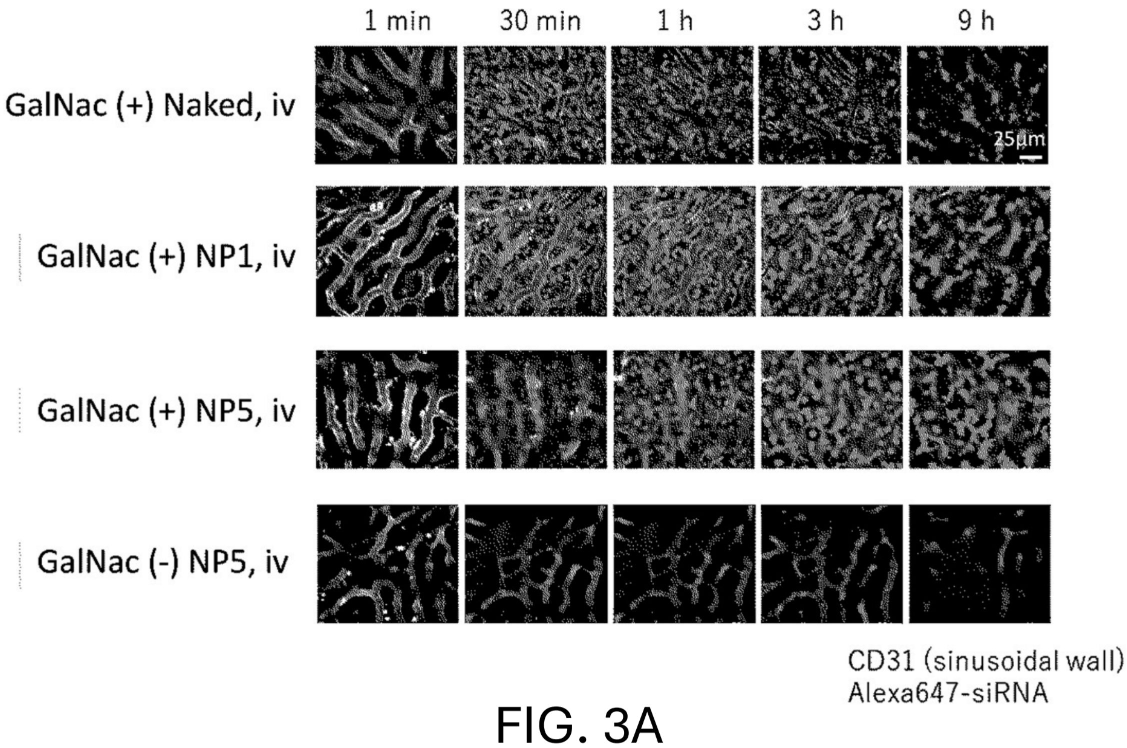
1 min
30 min
1 h
3 h
9 h
GalNac (+) Naked, iv
GalNac (+) NP1, iv
GalNac (+) NP5, iv
GalNac (-) NP5, iv
25μm
CD31 (sinusoidal wall)
Alexa647-siRNA

FIG. 3A

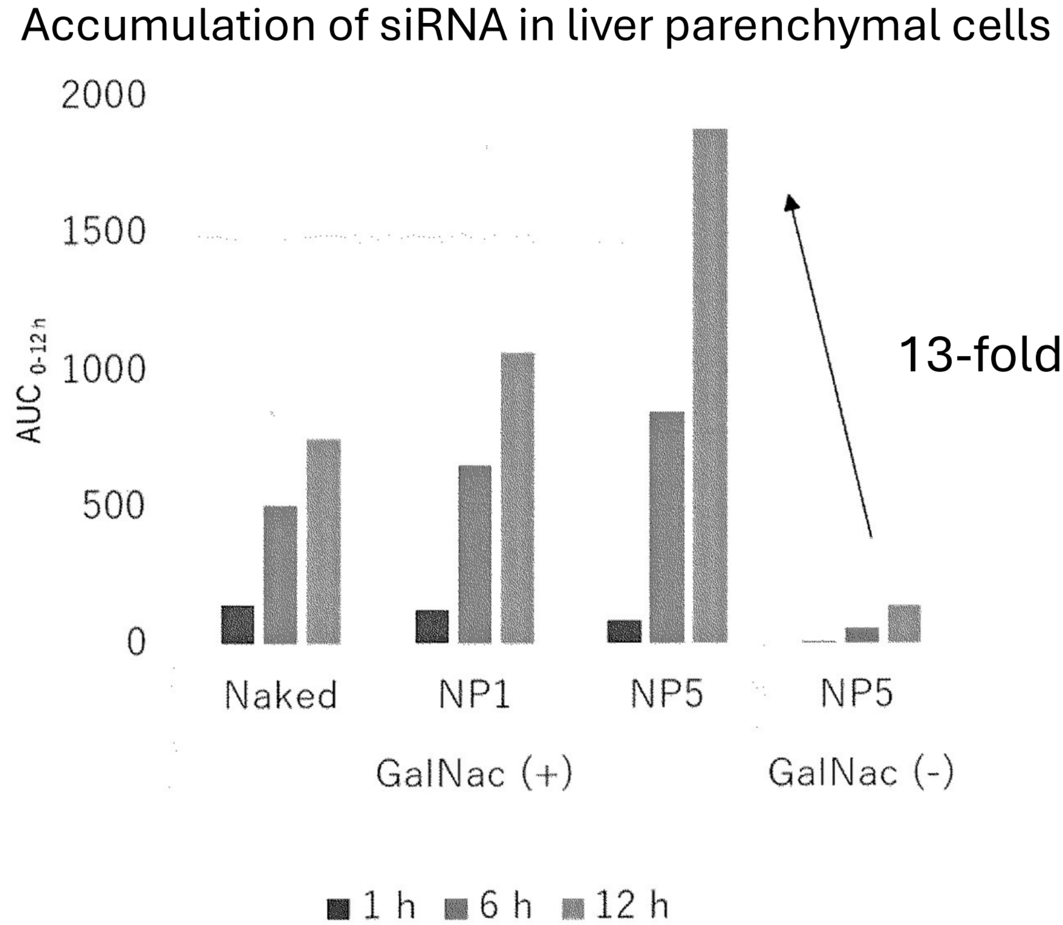
Accumulation of siRNA in liver parenchymal cells
2000
1500
1000
500
0
AUC 0-12 h
13-fold
Naked
NP1
NP5
NP5
GalNac (+)
GalNac (-)
1 h
6 h
12 h

FIG. 3B

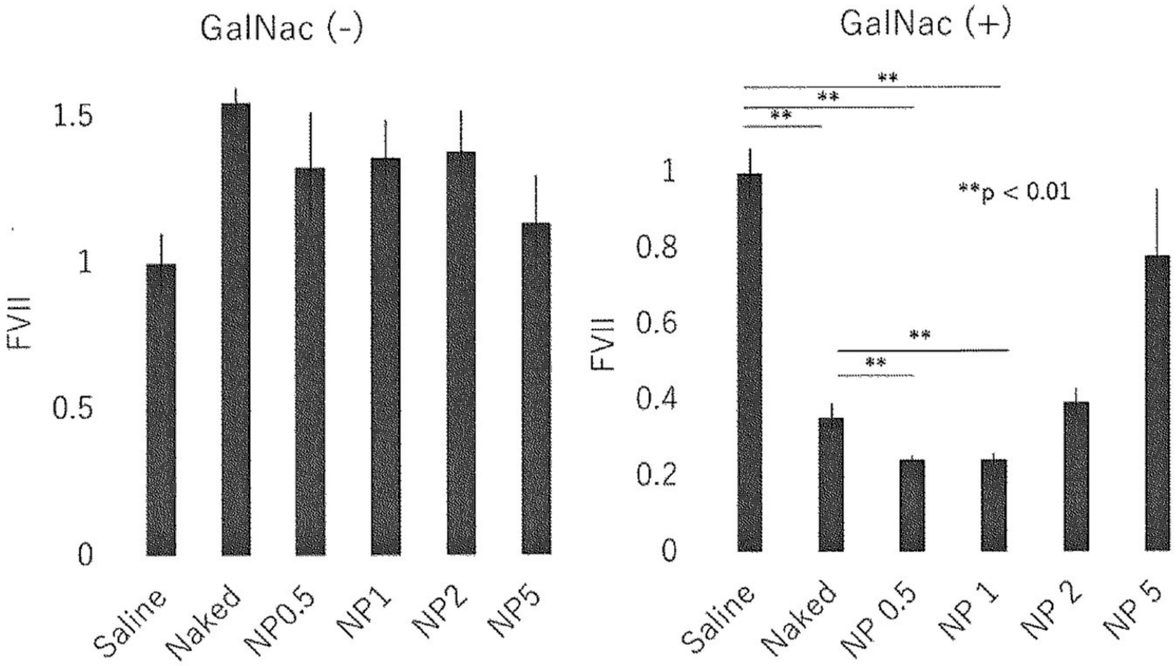
GalNac (-)
FVII
1.5
1
0.5
0
Saline
Naked
NP0.5
NP1
NP2
NP5
GalNac (+)
FVII
1
0.8
0.6
0.4
0.2
0
**
**p < 0.01
Saline
Naked
NP 0.5
NP 1
NP 2
NP 5

FIG. 4

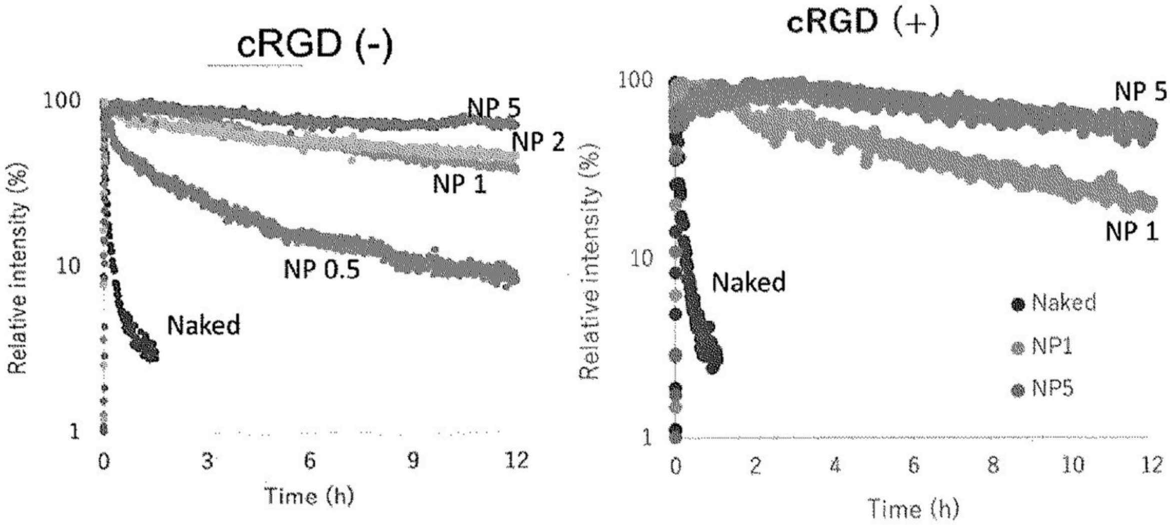
cRGD (-)
Relative intensity (%)
100
10
1
NP 5
NP 2
NP 1
NP 0.5
Naked
0
3
6
9
12
Time (h)
cRGD (+)
Relative intensity (%)
100
10
1
NP 5
NP 1
Naked
Naked
NP1
NP5
0
2
4
6
8
10
12
Time (h)

FIG. 5

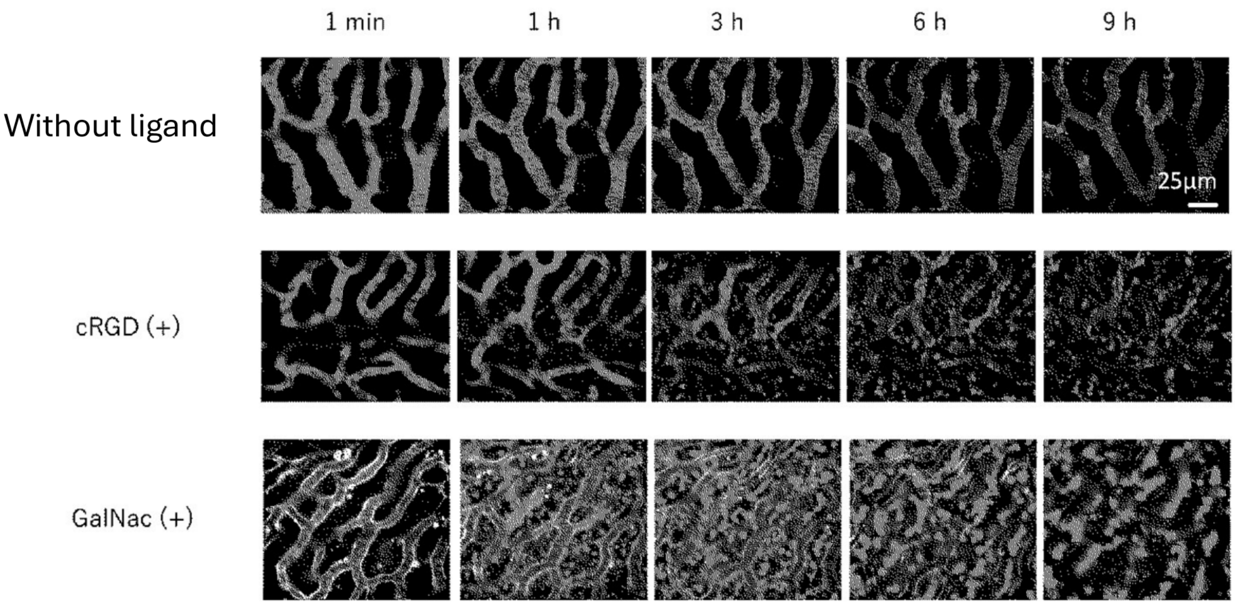


FIG. 6

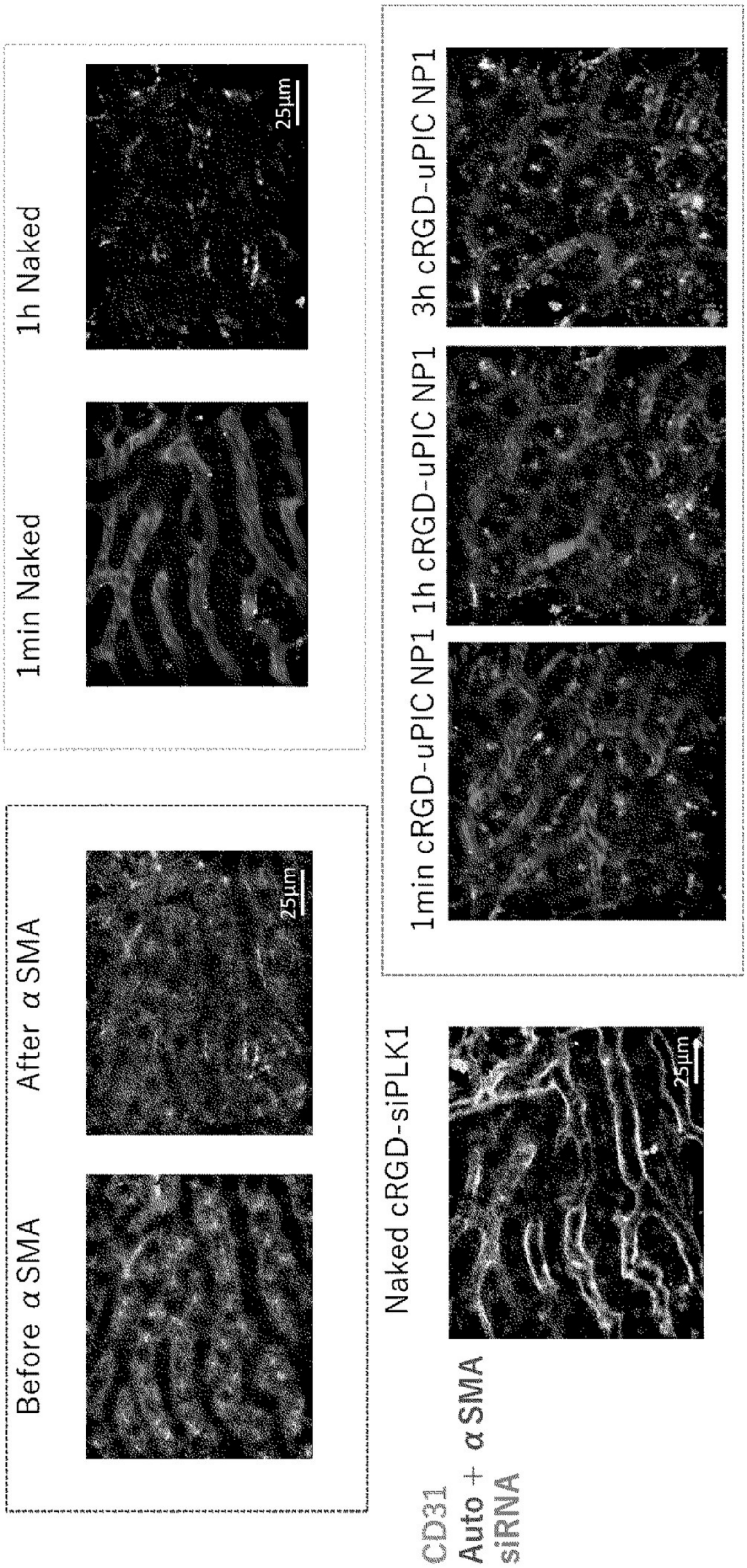
1min Naked
1h Naked
25µm
Before αSMA
After αSMA
25µm
1min cRGD-uPIC NP1
1h cRGD-uPIC NP1
3h cRGD-uPIC NP1
Naked cRGD-siPLK1
25µm
CD31
Auto + αSMA
siRNA

FIG. 7

## INTERNATIONAL SEARCH REPORT

International application No.
**PCT/JP2024/022479**

### A. CLASSIFICATION OF SUBJECT MATTER

***A61K 47/64***(2017.01)i; ***A61K 31/7088***(2006.01)i; ***A61K 47/26***(2006.01)i; ***A61K 47/34***(2017.01)i; ***A61K 47/36***(2006.01)i; ***A61K 47/42***(2017.01)i; ***A61K 47/61***(2017.01)i; ***A61P 43/00***(2006.01)i
FI: A61K47/64; A61K47/34; A61K47/26; A61K47/36; A61K47/42; A61K47/61; A61P43/00 111; A61P43/00 105; A61K31/7088 ZNA

According to International Patent Classification (IPC) or to both national classification and IPC

### B. FIELDS SEARCHED

Minimum documentation searched (classification system followed by classification symbols)

A61K47/64; A61K31/7088; A61K47/26; A61K47/34; A61K47/36; A61K47/42; A61K47/61; A61P43/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| Y | CHAYA, H. et al. Dynamic Stabilization of Unit Polyion Complexes Incorporating Small Interfering RNA by Fine-Tuning of Cationic Block Length in Two-Branched Poly(ethylene glycol)-b-poly(L-lysine). Biomacromolecules. 2022, vol. 23, no. 1, pp. 388-397<br>abstract, table 1, fig. 2 | 1-9 |
| Y | WO 2013/162041 A1 (THE UNIVERSITY OF TOKYO) 31 October 2013 (2013-10-31)<br>claims 1-2, 4, 7, paragraphs [0016], [0024], [0035], [0054], table 1, fig. 2 | 1-9 |
| Y | JP 2021-517909 A (DICERNA PHARMACEUTICALS, INC.) 29 July 2021 (2021-07-29)<br>paragraph [0054] | 1-9 |
| Y | JP 2005-512976 A (RIBOPHARMA AG) 12 May 2005 (2005-05-12)<br>claims 17-19, paragraph [0017] | 1-9 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **26 August 2024** | **03 September 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/022479**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/JP2024/022479**

| Patent document cited in search report | Publication date (day/month/year) | Patent family member(s) | Publication date (day/month/year) |
|---|---|---|---|
| WO 2013/162041 A1 | 31 October 2013 | US 2015/0080454 A1<br>claims 1-2, 4, 7, paragraphs [0029], [0038], [0062], [0091], table 1, fig. 2<br>EP 2842546 A1 | |
| JP 2021-517909 A | 29 July 2021 | US 2021/0010005 A1<br>paragraph [0072]<br>WO 2019/168686 A1<br>EP 3740248 A1<br>KR 10-2020-0127008 A<br>CN 112055597 A | |
| JP 2005-512976 A | 12 May 2005 | US 2008/0070856 A1<br>claim 36, paragraph [0018]<br>WO 2003/035083 A1<br>CN 1604783 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 8058069 B
- WO 2017212019 A
- WO 2019044937 A
- WO 2013162041 A
- WO 2019176916 A
- WO 2018216792 A
- US 9469664 B
- US 8278036 B


### Non-patent literature cited in the description


- **K. DEVANAND** ; **J. C. SELSER**. *Macromolecules*, 1991, vol. 24, 5943-5947